# EUROPÄISCHE PATENTSCHRIFT

(11) **EP 2 889 006 B1**
(45) Veröffentlichungstag und Bekanntmachung des Hinweises auf die Patenterteilung: **31.01.2018**
(21) Anmeldenummer: 14193616.1
(22) Anmeldetag: 18.11.2014
(51) Int. Cl.: A61B 17/02, A61B 17/28

(54) **Spreizer, insbesondere für die Schädelchirurgie**
Spreader, in particular for cranial surgery
Écarteur, en particulier pour la chirurgie crânienne

(30) Priorität: 18.11.2013 DE 202013105202 U
(43) Veröffentlichungstag der Anmeldung: 01.07.2015
(73) Patentinhaber: Fehling Instruments GmbH&Co. Kg, 63791 Karlstein (DE)
(72) Erfinder: Fehling, Guido, 63791 Karlstein (DE)
(74) Vertreter: Westphal, Mussgnug & Partner Patentanwälte mbB

(56) Entgegenhaltungen:
- WO-A2-01/50946
- US-A- 4 930 932
- US-A1- 2003 069 479
- US-A1- 2004 158 286
- US-B1- 6 168 601

## Beschreibung

Die Erfindung betrifft einen Spreizer, insbesondere für die Schädelchirurgie, gemäß dem Oberbegriff des Schutzanspruchs 1.

Bekannt sind Spreizer, insbesondere für die Schädelchirurgie, mit zwei um eine Drehachse gegeneinander verschwenkbaren Gelenkteilen, wobei jedes Gelenkteil am proximalen Ende ein Griffteil und am distalen Ende ein Spreizelement aufweist, wobei die Gelenkteile mit jeweils einem Abschnitt eine Ebene aufspannen und wobei an wenigstens einem der Gelenkteile ein Spannarm angeordnet ist, wobei der Spannarm mehrere Spannglieder aufweist, welche auf ein Zugseil gefädelt sind und welche abschnittsweise ineinander greifen und gegeneinander kippbar und/oder verdrehbar sind. Mit den Spreizelementen wird üblicherweise Weichgewebe aufgespreizt, während an den Spannarmen üblicherweise Hirnspatel befestigt werden, um Gewebe, insbesondere innerhalb des Schädels, in einer gewünschten Position zu halten.

Bei den bekannten Spreizern ist oft die Verbindung zwischen dem Spannarm und dem Gelenkteil nicht ausreichend fest und/oder für den gewählten Einsatz nicht im erforderlichen Umfang variabel.

Die Aufgabe der Erfindung besteht daher darin, einen Spreizer, insbesondere für die Schädelchirurgie, bereitzustellen, welcher flexibler und vorzugsweise sicherer einsetzbar ist.

Die Aufgabe der Erfindung wird gelöst durch einen Spreizer, insbesondere für die Schädelchirurgie, mit den Merkmalen des Patentanspruchs 1.

Vorteilhafte Ausgestaltungen und Weiterbildungen der Erfindungen sind in den unabhängigen Ansprüchen angegeben.

Der erfindungsgemäße Spreizer, insbesondere für die Schädelchirurgie, mit zwei um eine Drehachse gegeneinander verschwenkbaren Gelenkteilen, wobei jedes Gelenkteil am proximalen Ende ein Griffteil und am distalen Ende ein Spreizelement aufweist, wobei die Gelenkteile mit jeweils einem Abschnitt eine Ebene aufspannen und wobei an wenigstens einem der Gelenkteile ein Spannarm angeordnet ist, wobei der Spannarm mehrere Spannglieder aufweist, welche auf ein Zugseil gefädelt sind und welche abschnittsweise ineinander greifen und gegeneinander verkippbar und/oder verdrehbar sind, zeichnet sich dadurch aus, dass der Spannarm um eine Schwenkachse schwenkbar angeordnet ist und die Schwenkachse über einen in der Ebene angeordneten Arm mit dem Gelenkteil verbunden ist, wobei der Arm von dem Gelenkteil nach außen gerichtet ist, so dass die Schwenkachse in der Ebene zu dem Gelenkteil beabstandet ist.

Durch die Versetzung der Schwenkachse von dem Gelenkteil über den Arm nach außen wird es ermöglicht, durch geringere Verbiegung des Spannarms bzw. Anordnung des Spannarms in größeren Biegeradien als bei herkömmlichen Spreizern die gleichen Bereiche zwischen den beiden Gelenkteilen mit dem distalen Ende des Spannarms zu erreichen. Dadurch, dass der Spannarm weniger verbogen werden muss bzw. mit größeren Biegeradien verwendet werden kann, wird der Spannvorgang erleichtert und eine stabile Fixierung des Spannarms ermöglicht. Insbesondere ermöglicht der vergrößerte Abstand zwischen der Schwenkachse, an welcher der Spannarm befestigt ist, und der Drehachse zwischen den beiden Gelenkteilen eine hindernisfreie Positionierung und Kontraktion des entsprechenden Spannarms.

Vorteilhafterweise ist der Arm starr, insbesondere einstückig, an dem Gelenkteil angeordnet.

Vorteilhafterweise weisen die Spannglieder eine Länge und einen Durchmesser auf, wobei das Verhältnis von Durchmesser zu Länge im Bereich von 1:1 bis 2:3 liegt. Die Spannglieder können somit kürzer als herkömmlicherweise ausgebildet werden, was eine flexiblere Anordnung des Spannarms ermöglicht.

Eine vorteilhafte Ausgestaltung sieht vor, dass die Spannglieder jeweils einen zylindrischen Abschnitt und einen kugelsegmentförmigen Abschnitt aufweisen, wobei ein kugelsegmentförmiger Abschnitt eines Spannglieds in einen zylindrischen Abschnitt eines anderen Spannglieds eingreift. Dadurch wird auch bei Krümmung des Spannarms eine möglichst glatte oder nahezu geschlossen verlaufende Oberfläche des Spannarms erreicht, welche insbesondere kaum Kanten oder Ecken aufweist, die ein Verletzungsrisiko bergen würden.

Gemäß einer besonders bevorzugten Ausführungsform der Erfindung weisen die Spannglieder wenigstens ein, vorzugsweise mehrere, Fenster auf, welches vorzugsweise in dem zylindrischen Abschnitt angeordnet sind. Dies ermöglicht eine gute Reinigung der Spannglieder des Spannarms.

Besonders bevorzugt weist der Spannarm einen um die Schwenkachse verlaufenden Zahnkranz auf, welcher auf einen an dem Arm angeordneten um die Schwenkachse verlaufenden Zahnkranz aufsetzbar ist. Diese ineinandergreifenden Zahnkränze ermöglichen eine stabile, insbesondere winkelstabile, Positionierung des Spannarms an dem Gelenkteil in einer gewünschten Position und verhindern ein unerwünschtes Verdrehen des Spannarms um die Schwenkachse. Andererseits ermöglichen die Zahnkränze eine Positionierung des Spannarms in verschiedenen um die Schwenkachse verdrehten Winkelstellungen relativ zu dem an dem Arm angeordneten Zahnkranz, was einen flexiblen Einsatz ermöglicht. Bei feiner Zahnung wird insbesondere eine nahezu beliebige Winkelverbindung zwischen dem Spannarm und dem entsprechenden Gelenkteil ermöglicht. Zudem ermöglichen die beiden Zahnkränze eine platzsparende Verbindung, da beispielsweise die beiden Zahnkränze durch eine zentral verlaufende Bohrung miteinander verschraubt werden können.

Vorteilhafterweise weist der Spannarm zwei parallel zueinander angeordnete um die Schwenkachse verlaufende Zahnkränze auf, deren Zähne in entgegengesetzter Richtung weisen, was den flexiblen Einsatz des Spannarms weiter vergrößert, insbesondere derart, dass der Spannarm an beiden Gelenkteilen des Spreizers eingesetzt werden kann.

Vorzugsweise ist am distalen Ende des Spannarms ein Halter für einen Hirnspatel angeordnet. Dies vergrößert die Flexibilität des erfindungsgemäßen Spreizers weiter.

Vorzugsweise ist das distale Ende des Spannarms schräg abgeschnitten und der Halter mit einer Anlagefläche auf der schräg abgeschnittenen Fläche des distalen Endes des Spannarms angeordnet, entweder starr verbunden angeordnet oder um eine Drehachse, welche die schräg abgeschnittene Fläche bzw. die Anlagefläche senkrecht schneidet, drehbar gelagert angeordnet. Bei Drehung des Halters um das schräg abgeschnittene distale Ende des Spannarms wird somit eine Ausrichtung eines in dem Halter angeordneten Spreizelements wie beispielsweise eines Hirnspatels in verschiedenen Raumrichtungen ermöglicht.

Eine bevorzugte Ausführungsform der Erfindung sieht vor, dass der Hirnspatel aus einem Formgedächtnismaterial, beispielsweise Nitinol, gefertigt ist, welches insbesondere bei Raumtemperatur beliebig dem chirurgischen Zweck angeformt werden kann und bei der Aufbereitung unter dem thermischen Einfluss von heißem Spülwasser seine Ausgangsform zurückgewinnt, was eine Verbesserung der flexiblen Einsatzmöglichkeiten und der zweckorientierten Handlichkeit des Spreizers sowie eine Verlängerung der Lebensdauer der Hirnspatel ermöglicht.

Vorzugsweise ist zwischen den beiden Gelenkteilen, insbesondere zwischen den beiden Griffteilen, ein Sperrmechanismus, vorzugsweise eine Ratschsperre angeordnet, um ein Fixieren der beiden Gelenkteile in einer gewünschten Winkelstellung relativ zueinander zu ermöglichen.

Vorteilhafterweise ist das Spreizelement als Spreizgabel ausgebildet und bevorzugt aus der Ebene abgewinkelt angeordnet, um ein zuverlässiges Zurückhalten von Gewebe zu ermöglichen.

Die Erfindung wird anhand der nachfolgenden Figuren ausführlich erläutert. Es zeigen
- Fig. 1a: eine perspektivische Ansicht eines Ausführungsbeispiels eines erfindungsgemäßen Spreizers mit daran angeordneten Spannarmen,
- Fig. 1b: eine Draufsicht auf die Anordnung gemäß Fig. 1a,
- Fig. 1c: eine Seitenansicht der Anordnung gemäß Fig. 1a,
- Fig. 1d: eine Frontansicht der Anordnung gemäß Fig. 1a,
- Fig. 2a: eine perspektivische Ansicht des Spreizers gemäß Fig. 1a ohne daran angeordnete Spannarme,
- Fig. 2b: eine Draufsicht auf den Spreizer gemäß Fig. 2a,
- Fig. 2c: eine Seitenansicht des Spreizers gemäß Fig. 2a,
- Fig. 3a: eine perspektivische Ansicht eines Spannarms des Spreizers gemäß Fig. 1a,
- Fig. 3b: eine Draufsicht auf den Spannarm gemäß Fig. 3a und
- Fig. 3c: eine Seitenansicht des Spannarms gemäß Fig. 3a.

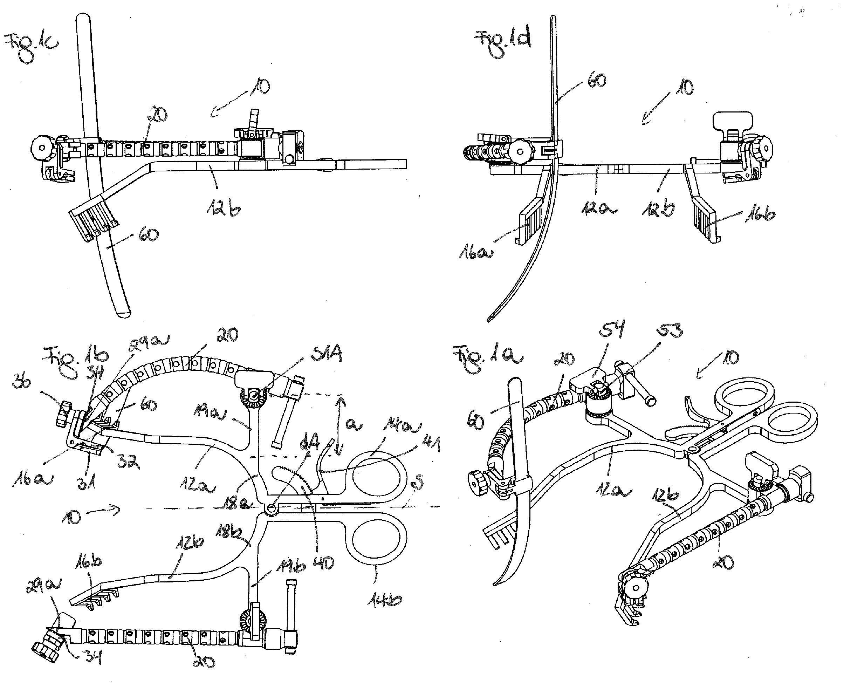

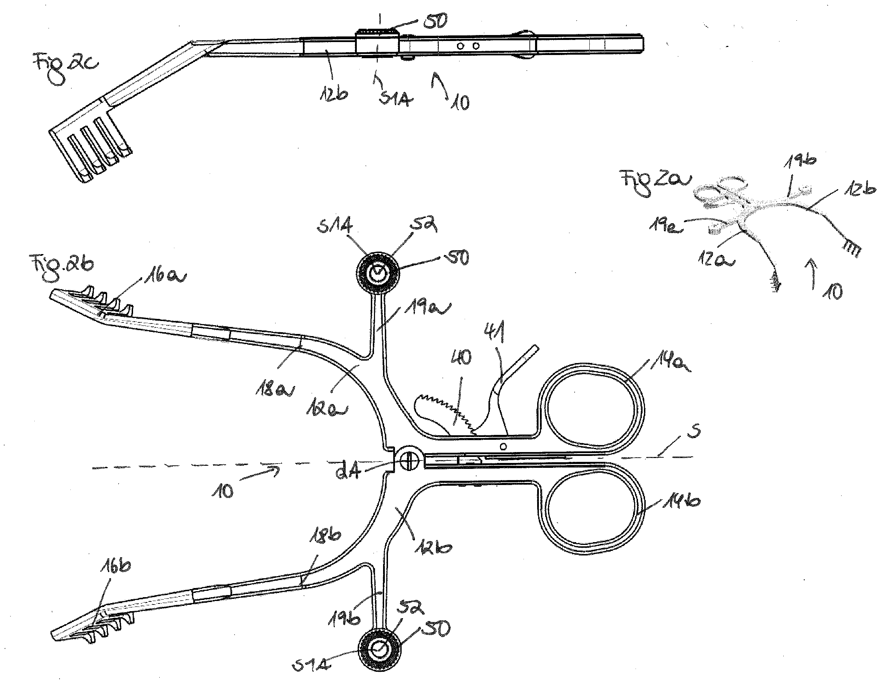

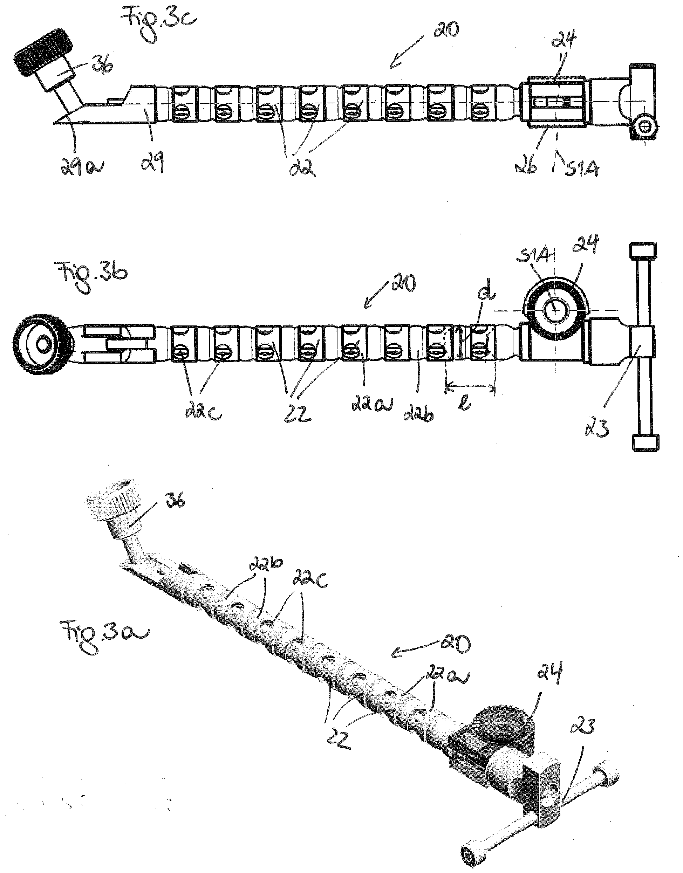

Die Figuren 1a bis 1d zeigen ein Ausführungsbeispiel eines erfindungsgemäßen Spreizers 10 mit zwei daran angeordneten Spannarmen 20, die Figuren 2a bis 2c zeigen den Spreizer 10 gemäß der Figuren 1a bis 1d ohne die daran angeordneten Spannarme 20 und die Figuren 3a bis 3c zeigen verschiedene Ansichten der Spannarme 20 des Spreizers gemäß der Figuren 1a bis 1d.

Der Spreizer 10 weist zwei um eine Drehachse dA schwenkbar gegeneinander angeordnete Gelenkteile 12a, 12b auf, welche jeweils ein Griffteil 14a, 14b am proximalen Ende sowie am distalen Ende ein Spreizelement 16a, 16b aufweisen. Dabei sind die Gelenkteile 12a, 12b insbesondere derart zueinander angeordnet, dass sie sich nicht kreuzen, so dass bei Schließen der beiden Gelenkteile 12a, 12b gegeneinander die Spreizelemente 16a, 16b auseinandergedrückt werden. Zwischen den Griffteilen 14a, 14b und den Spreizelementen 16a, 16b ist ein Abschnitt 18a, 18b angeordnet, welche eine Ebene aufspannen. Die Griffteile 14a, 14b liegen insbesondere ebenfalls in dieser Ebene.

Die Spreizelemente 16a, 16b sind in einer Ausführungsform aus der Ebene abgewinkelt. Die Spreizelemente 16a, 16b können insbesondere als Spreizgabeln ausgebildet sein.

Zwischen den beiden Gelenkteilen 12a, 12b, insbesondere durch die Drehachse dA und parallel zu den zwischen der Drehachse dA und den Griffteilen 14a, 14b verlaufenen Abschnitten der Gelenkteile 12a, 12b kann insbesondere eine Symmetrieachse S gebildet sein.

Zwischen den beiden Gelenkteilen 12a, 12b, insbesondere zwischen den beiden Griffteilen 14a, 14b, ist eine Ratschsperre 40 angeordnet, mittels welcher die beiden Gelenkteile 12a, 12b in einer relativen Position zueinander fixiert werden können. Die Ratschsperre 40 kann mittels eines schwenkbar gelagerten Lösehebels 41 gelöst werden.

Während die Gelenkteile 12a, 12b im Bereich zwischen der Drehachse dA und den Griffteilen 14a, 14b im Wesentlichen parallel zueinander angeordnet sind, verlaufen sie im Bereich zwischen der Drehachse dA und den Spreizelementen 16a, 16b bogenförmig und bilden insbesondere gemeinsam ein U. Bei Verwendung des Spreizers 10 versperren somit die Gelenkteile 12a, 12b nicht die Sicht auf das Operationsgebiet.

An wenigstens einem, in der vorliegenden Ausführungsform an beiden, der Gelenkteile 12a, 12b ist zwischen den Griffteilen 14a, 14b und den Spreizelementen 16a, 16b, insbesondere im Bereich zwischen der Drehachse dA und den Spreizelementen 16a, 16b, vorzugsweise jedoch näher an der Drehachse dA als an den Spreizelementen 16a, 16b, ein Arm 19a, 19b derart angeordnet, dass er nach außen gerichtet ist und somit insbesondere von dem jeweils anderen Gelenkteil 12b, 12a wegweist. Die Arme 19a, 19b liegen in der durch die Abschnitte 18a, 18b aufgespannten Ebene. Vorteilhafterweise liegen beide Arme 19a, 19b auf einer Geraden, welche insbesondere senkrecht zur Symmetrieachse S des Spreizers 10 angeordnet ist. Die Arme 19a, 19b sind insbesondere starr, vorteilhafterweise einstückig, an den jeweiligen Gelenkteilen 12a, 12b angeordnet.

An den freien Enden der Arme 19a, 19b ist eine Vorrichtung zur Befestigung jeweils eines Spannarms 20 angeordnet.

Die Befestigung des Spannarms 20 erfolgt derart, dass der Spannarm 20 um eine Schwenkachse S1A drehbar gelagert an dem Arm 19a, 19b angeordnet ist. Die Schwenkachse S1A ist dabei in der Ebene um einen Abstand a, welcher etwa der Länge des Arms 19a, 19b entspricht, von dem Gelenkteil 12a, 12b beabstandet. Dadurch wird der Befestigungspunkt des Spannarms 20 weiter nach außen verlagert, was größere Krümmungsradien der Spannarme 20 zum Erreichen einer gewünschten Position innerhalb des U-förmigen Bereichs zwischen den Gelenkteilen 12a, 12b sowie eine Verkürzung der Spannarme 20 ermöglicht.

Zur Befestigung des Spannarms ist insbesondere an dem Arm 19a, 19b ein Zahnkranz 50 mit auf der Stirnseite radial nach außen verlaufenden Zähnen angeordnet. In dem Zahnkranz 50 ist axial eine Bohrung 52 angeordnet, welche insbesondere ein Innengewinde aufweist.

Der Spannarm 20 weist einen ersten Zahnkranz 24 auf, welcher an seiner Stirnseite radial nach außen verlaufende Zähne aufweist und auf den Zahnkranz 50 aufsetzbar ist. Dabei weist der Zahnkranz 24 insbesondere eine axiale Bohrung 25 auf, welche mit der Bohrung 52 des Zahnkranzes 50 der Arme 19a, 19b fluchtend angeordnet ist. Die beiden Zahnkränze 24, 50 können in verschiedenen Winkelstellungen relativ zueinander aufeinander gesetzt werden, so dass ein flexible Ausrichtung des Spannarms 20 möglich ist.

Eine Befestigung des Spannarms 20 kann dadurch erfolgen, dass eine Schraube 53 durch die Bohrung 25 und die Bohrung 52 geführt und beispielsweise in der Bohrung 52 verschraubt wird. Um die Schraube 53 ohne weitere Hilfsmittel eindrehen zu können, weist diese an ihrem Kopf vorzugsweise einen Hebel 54 auf, welcher das Eindrehen der Schraube 53 erleichtert und vorteilhafterweise um eine Achse schwenkbar angeordnet ist, um den Hebel 54 abzuklappen und platzsparend anzuordnen, sofern er nicht benötigt wird.

Der Spannarm 20 kann neben dem ersten Zahnkranz 24 einen zweiten Zahnkranz 26 aufweisen, welcher parallel zu dem ersten Zahnkranz 24 angeordnet ist und an seiner Stirnseite radial verlaufende Zähne aufweist, die in entgegengesetzte Richtung zu der Richtung der Zähne des ersten Zahnkranzes 24 weisen. Dies ermöglicht einerseits ein um 180° um die Längsachse des Spannarms 20 gedrehtes Aufsetzen des Spannarms 20 an einen Arm 19a, 19b, andererseits jedoch auch das wechselseitige Anordnen eines Spannarms 20 sowohl an dem einen Arm 19a als auch an dem anderen Arm 19b. Die Zahnung der Zahnkränze 50, 24, 26 ist dabei derart fein gewählt, dass bei Verdrehung der Zahnkränze 24, 26 gegen den Zahnkranz 50 um einen Zahn nur eine Verdrehung um wenige Winkelgrade erfolgt.

Der Spannarm 20 weist mehrere Spannglieder 22 auf, welche jeweils einen zylindrischen Abschnitt 22a und einen kugelsegmentförmigen Abschnitt 22b aufweisen können. Dabei greift der kugelige Abschnitt 22b eines der Spannglieder 22 in den zylindrischen Abschnitt 22a eines anderen der Spannglieder 22 ein. Der kugelsegmentförmige Abschnitt 22b ermöglicht dabei ein Verkippen der aneinander grenzenden Spannglieder 22 in beliebiger Richtung sowie ein Verdrehen der aneinandergrenzenden Spannglieder 22 um ihre durch den zylindrischen Abschnitt 22a verlaufende Längsachse. Die Spannglieder 22 sind auf ein nicht dargestelltes Zugseil aufgefädelt, welches an einem distalen Spannglied 29, welches das distale Ende des Spannarms 20 bildet, einendseitig befestigt ist, während das andere Ende am proximalen Ende des Spannarms 20 an einer Spannvorrichtung 23 befestigt ist. Um die Spannglieder 22 auffädeln zu können, weisen sie eine durchgehende Längsöffnung auf und sind insbesondere als Hohlkörper ausgebildet. Die Spannvorrichtung 23 weist insbesondere einen Schwenkhebel auf, mit welcher bei Verschwenken die in dem Spannarm 20 verbleibende Länge des Zugseils verkürzt und auf diese Weise der Spannarm 20 gespannt und die Spannglieder 22 in ihrer relativen Position gegeneinander fixiert werden. In einer Ausführungsform weisen die Spannglieder 22 mindestens ein, beispielsweise vier äquidistant über den Umfang verteilte, Fenster 22c auf, insbesondere in dem zylindrischen Abschnitt 22a, welche ein einfaches Reinigen der Spannglieder 22 ermöglichen. Die Spannglieder 22 weisen eine Länge 1 und einen Durchmesser d auf, wobei die Länge 1 beispielsweise 12 mm und der Durchmesser d beispielsweise 8 mm betragen kann.

Am distalen Ende des Spannarms 20, insbesondere an dem distalen Spannglied 29, kann ein Halter 30 für einen Hirnspatel 60 angeordnet sein. Der Halter 30 weist zwei Klemmbacken 31, 32 auf, zwischen welchen der Hirnspatel 60 klemmend, beispielsweise federbeaufschlagt oder nach Anziehen einer Feststellschraube 36, gehalten werden kann. Der Halter 30 weist eine Anlagefläche 34 auf, mit welcher er an dem distalen Ende des Spannarms 20 anliegt.

In einer Ausführungsform ist das distale Ende des Spannarms 20 schräg abgeschnitten, beispielsweise in einem Winkel von 45° zur Längsachse des distalen Spannglieds 29. Die Anlagefläche 34 des Halters 30 kann starr verbunden an dem distalen Ende des Spannarms 20 oder alternativ um eine Achse, welche senkrecht zu der Anlagefläche 34 verläuft, drehbar gelagert an dem Spannarm 20 angeordnet sein. Bei Drehung des Halters 30 um diese Achse kann der zwischen den Klemmbacken 31, 32 gehaltene Hirnspatel 60 in verschiedene Raumrichtungen ausgerichtet werden. Die Feststellschraube 36 kann die Schwenkachse bilden und nach Festlegen der gewünschten Ausrichtung zwischen dem Halter 30 und dem distalen Ende des Spannarms 20 durch Festziehen sowohl den Halter 30 an dem distalen Ende des Spannarms 20 in der gewünschten Position als auch gleichzeitig den Hirnspatel 30 zwischen den beiden Klemmbacken 31, 32 fixieren. Die Klemmbacken 31, 32 können rechtwinklig abgebogen sein, was einen kompakteren Aufbau ermöglicht.

Der Hirnspatel 60 kann aus einem Formgedächtnismaterial, beispielsweise Nitinol, gefertigt sein, um einen flexiblen Einsatz während der Operation und gleichzeitig eine Wiederverwendbarkeit nach Reinigung zu ermöglichen.

### Bezugszeichenliste

- 10: Spreizer
- 12a, b: Gelenkteil
- 14a, b: Griffteil
- 16a, b: Spreizelement
- 18a, b: Abschnitt
- 19a, b: Arm

- 20: Spannarm
- 22: Spannglied
- 22a: zylindrischer Abschnitt
- 22b: kugelsegmentförmiger Abschnitt
- 22c: Fenster
- 23: Spannvorrichtung
- 24: erster Zahnkranz
- 26: zweiter Zahnkranz
- 29: distales Spannglied
- 29a: schräge Fläche

- 30: Halter
- 31: Klemmbacke
- 32: Klemmbacke
- 34: Anlagefläche
- 36: Feststellschraube

- 40: Ratschsperre
- 41: Lösehebel

- 50: Zahnkranz
- 52: Bohrung
- 53: Schraube
- 54: Hebel
- 60: Hirnspatel

- dA: Drehachse
- S1A: Schwenkachse
- a: Abstand
- l: Länge
- d: Durchmesser
- S: Symmetrieachse

## Patentansprüche

1. Spreizer (10) insbesondere für die Schädelchirugie, mit zwei um eine Drehachse (dA) gegeneinander verschwenkbaren Gelenkteilen (12a, 12b), wobei jedes Gelenkteil (12a, 12b) am proximalen Ende ein Griffteil (14a, 14b) und am distalen Ende ein Spreizelement (16a, 16b) aufweist, wobei die Gelenkteile (12a, 12b) mit jeweils einem Abschnitt (18a, 18b) eine Ebene aufspannen und wobei an wenigstens einem der Gelenkteile (12a, 12b) ein Spannarm (20) angeordnet ist, wobei der Spannarm (20) mehrere Spannglieder (22) aufweist, welche auf ein Zugseil gefädelt sind und welche abschnittsweise ineinandergreifen und gegeneinander verkippbar und/oder verdrehbar sind,
**dadurch gekennzeichnet, dass** der Spannarm (20) um eine Schwenkachse (S1A) schwenkbar angeordnet ist und die Schwenkachse (S1A) über einen in der Ebene angeordneten Arm (19a, 19b) mit dem Gelenkteil (12a, 12b) verbunden ist, wobei der Arm (19a, 19b) von dem Gelenkteil (12a, 12b) nach außen gerichtet ist, so dass die Schwenkachse (S1A) in der Ebene zu dem Gelenkteil (12a, 12b) beabstandet ist.

2. Spreizer (10) nach Anspruch 1,
**dadurch gekennzeichnet, dass** die Spannglieder (22) eine Länge (1) und einen Durchmesser (d) aufweisen, wobei das Verhältnis von Durchmesser (d) zu Länge (1) im Bereich von 1:1 bis 2:3 liegt.

3. Spreizer (10) nach einem der vorhergehenden Ansprüche,
**dadurch gekennzeichnet, dass** die Spannglieder (22) jeweils einen zylindrischen Abschnitt (22a) und einen kugelsegmentförmigen Abschnitt (22b) aufweisen, wobei ein kugelsegmentförmiger Abschnitt (22b) eines Spannglieds (22) in einen zylindrischen Abschnitt (22a) eines anderen Spannglieds (22) eingreift.

4. Spreizer (10) nach einem der vorhergehenden Ansprüche,
**dadurch gekennzeichnet, dass** die Spannglieder (22) wenigstens ein, vorzugsweise mehrere, Fenster (22c) aufweisen, welche vorzugsweise in dem zylindrischen Abschnitt (22a) angeordnet sind.

5. Spreizer (10) nach einem der vorhergehenden Ansprüche,
**dadurch gekennzeichnet, dass** der Spannarm (22) einen um die Schwenkachse (S1A) verlaufenden Zahnkranz (24) aufweist, welcher auf einen an dem Arm (19a, 19b) angeordneten um die Schwenkachse (S1A) verlaufenden Zahnkranz (50) aufsetzbar ist.

6. Spreizer (10) nach einem der vorhergehenden Ansprüche,
**dadurch gekennzeichnet, dass** der Spannarm (22) zwei parallel zueinander angeordnete um die Schwenkachse (S1A) verlaufende Zahnkränze (24, 26) aufweist, deren Zähne in entgegengesetzte Richtung weisen.

7. Spreizer (10) nach einem der vorhergehenden Ansprüche,
**dadurch gekennzeichnet, dass** am distalen Ende des Spannarms (20) ein Halter (30) für einen Hirnspatel (60) angeordnet ist.

8. Spreizer (10) nach einem der vorhergehenden Ansprüche,
**dadurch gekennzeichnet, dass** das distale Ende des Spannarms (20) schräg abgeschnitten ist und der Halter (30) mit einer Anlagefläche (34) an der schräg abgeschnittenen Fläche des distalen Endes des Spannarms (20) anliegt und um eine Drehachse, welche die Anlagefläche (30) senkrecht schneidet, drehbar gelagert angeordnet ist.

9. Spreizer (10) nach einem der vorhergehenden Ansprüche,
**dadurch gekennzeichnet, dass** der Hirnspatel (60) aus einem Formgedächtnismaterial, beispielsweise Nitinol, gefertigt ist.

10. Spreizer (10) nach einem der vorhergehenden Ansprüche,
**dadurch gekennzeichnet, dass** zwischen den beiden Gelenkteilen (12a, 12b), insbesondere zwischen den beiden Griffteilen (14a, 14b), ein Sperrmechanismus, vorzugsweise eine Ratschsperre (40), angeordnet ist.

11. Spreizer (10) nach einem der vorhergehenden Ansprüche,
**dadurch gekennzeichnet, dass** das Spreizelement (16a, 16b) als Spreizgabel ausgebildet ist und vorzugsweise aus der Ebene abgewinkelt angeordnet ist.

## Claims

1. Retractor (10), in particular for cranial surgery, comprising two articulated parts (12a, 12b) which are pivotable relative to one another about an axis of rotation (dA), wherein each articulated part (12a, 12b) has a handle part (14a, 14b) on the proximal end and a retracting element (16a, 16b) on the distal end, wherein at least a section (18a, 18b) of each articulated part (12a, 12b) spans a plane and wherein a clamping arm (20) is arranged on at least one of the articulated parts (12a, 12b), wherein the clamping arm (20) has a plurality of tendons (22), which are threaded onto a traction cable and which engage with one another in sections and can be tilted and/or rotated relative to one another, **characterised in that** the clamping arm (20) is arranged pivotably about a pivot axis (S1A) and the pivot axis (S1A) is connected to the articulated part (12a, 12b) by means of an arm (19a, 19b) arranged in the plane, wherein the arm (19a, 19b) is directed outwards from the articulated part (12a, 12b), so that the pivot axis (S1A) is spaced apart from the articulated part (12a, 12b) in the plane.

2. Retractor (10) according to claim 1, **characterised in that** the tendons (22) have a length (I) and a diameter (d), wherein the ratio of the diameter (d) to the length (I) is in the range from 1:1 to 2:3.

3. Retractor (10) according to one of the preceding claims, **characterised in that** the tendons (22) each have a cylindrical section (22a) and a semi-spherical section (22b), wherein a semi-spherical section (22b) of a tendon (22) engages in a cylindrical section (22a) of another tendon (22).

4. Retractor (10) according to one of the preceding claims, **characterised in that** the tendons (22) have at least one, preferably a plurality of windows (22c) which are preferably arranged in the cylindrical section (22a).

5. Retractor (10) according to one of the preceding claims, **characterised in that** the clamping arm (22) has a ring gear (24) which runs around the pivot axis (S1A) and can be fitted onto a ring gear (50) which is arranged on the arm (19a, 19b) and runs around the pivot axis (S1A).

6. Retractor (10) according to one of the preceding claims, **characterised in that** the clamping arm (22) has two ring gears (24, 26) which are arranged parallel to one another and run around the pivot axis (S1A), and the teeth of the ring gears point in opposite directions.

7. Retractor (10) according to one of the preceding claims, **characterised in that** a holder (30) for a brain spatula (60) is arranged on the distal end of the clamping arm (20).

8. Retractor (10) according to one of the preceding claims, **characterised in that** the distal end of the clamping arm (20) is bevelled and a bearing surface (34) of the holder (30) bears against the bevelled surface of the distal end of the clamping arm (20) and is arranged so as to be mounted rotatably about an axis of rotation which vertically intersects the bearing surface (30).

9. Retractor (10) according to one of the preceding claims, **characterised in that** the brain spatula (60) is made from a shape memory material, for example nitinol.

10. Retractor (10) according to one of the preceding claims, **characterised in that** a locking mechanism, preferably a ratchet lock (40), is arranged between the two articulated parts (12a, 12b), in particular between the two handle parts (14a, 14b).

11. Retractor (10) according to one of the preceding claims, **characterised in that** the retracting element (16a, 16b) is configured as a retracting fork and is preferably arranged at an angle to the plane.

## Revendications

1. Ecarteur (10) en particulier pour la chirurgie crânienne comprenant deux pièces articulées (12a, 12b) susceptibles de pivoter l'une par rappeur à l'autre autour d'un axe de rotation (dA), chacune de ces pièces articulées (12a, 12b) comportant, à son extrémité proximale une pièce de préhension (14a, 14b) et à son extrémité distale un élément d'écartement (16a, 16b), les pièces articulées (12a, 12b) couvrant un plan avec un segment respectif (18a, 18b), et, un bras tendeur (20) étant monté sur au moins l'une des pièces articulées (12a, 12b), le bras tendeur (20) comportant plusieurs organes tendeurs (22) qui sont enfilés sur un câble de traction, et viennent en prise par segments les uns dans les autres sont susceptibles de s'incliner ou de tourner les uns par rapport aux autres,
**caractérisé en ce que**
le bras tendeur (20) est monté pivotant autour d'un axe de pivotement (S1A) et l'axe de pivotement (S1A) est relié à la pièce articulée (12a, 12b), par l'intermédiaire d'un bras (19a, 19b) situé dans le plan le bras (19a, 19b) étant orienté vers l'extérieur à partir de la pièce articulée (12a, 12b) de sorte que l'axe de pivotement (S1A) soit situé à distance de la pièce articulée (12a, 12b) dans le plan.

2. Ecarteur (10) conforme à la revendication1,
**caractérisé en ce que**
les organes tendeurs (22) ont une longueur (l) et un diamètre (d), le rapport du diamètre (d) à la longueur (l) étant situé dans la plage de 1:1 à 2:3.

3. Ecarteur (10) conforme à l'une des revendications précédentes,
**caractérisé en ce que**
les organes tendeurs (22) comportent chacun un segment cylindrique (22a) et un segment en forme de segment sphérique (22b), un segment (22b) en forme de segment sphérique d'un organe tendeur (22) venant en prise dans un segment cylindrique (22a) d'un autre organe tendeur (22).

4. Ecarteur (10) conforme à l'une des revendications précédentes,
**caractérisé en ce que**
les organes tendeurs (22) comportent au moins une et de préférence plusieurs fenêtre(s) (22c) qui est(sont) de préférences située(s) dans le segment cylindrique (22a).

5. Ecarteur (10) conforme à l'une des revendications précédentes,
**caractérisé en ce que**
le bras tendeur (22) comporte une couronne dentée (24) s'étendant autour de l'axe de pivotement (S1A), et qui peut être positionnée sur une couronne dentée (50) montée sur le bras (19a, 19b) s'étendant autour de l'axe de pivotement (S1A).

6. Ecarteur (10) conforme à l'une des revendications précédentes,
**caractérisé en ce que**
le bras tendeur (22) comporte deux couronnes dentées (24, 26) montées parallèlement s'étendant autour de l'axe de rotation (S1A) et, dont les dents présentent des directions opposées.

7. Ecarteur (10) conforme à l'une des revendications précédentes,
**caractérisé en ce qu'**
à l'extrémité distale du bras tendeur (20) est monté un élément support (30) pour spatule cérébrale (60).

8. Ecarteur (10) conforme à l'une des revendications précédentes,
**caractérisé en ce que**
l'extrémité distale du bras tendeur (20) est découpée en biais et l'élément support (30) s'applique, par une surface d'appui (34) sur la surface découpée en biais de l'extrémité distale du bras tendeur (20), et est monté mobile en rotation autour d'un axe de rotation qui coupe perpendiculairement la surface d'appui (30).

9. Ecarteur (10) conforme à l'une des revendications précédentes,
**caractérisé en ce que**
la spatule cérébrale (60) est réalisée en un matériau à mémoire de forme, par exemple en nitinol.

10. Ecarteur (10) conforme à l'une des revendications précédentes,
**caractérisé en ce qu'**
entre les deux pièces articulées (12a, 12b), en particulier entre les deux pièces de préhension (14a, 14b) est monté un mécanisme de blocage, de préférence un verrou à cliquet (40).

11. Ecarteur (10) conforme à l'une des revendications précédentes,
**caractérisé en ce que**
l'élément d'écartement (16a, 16b) est réalisé sous la forme d'une fourchette d'écartement et est de préférence coudé en dehors du plan.
